(19) Europäisches Patentamt
European Patent Office
Office européen des brevets

(11) **EP 4 410 943 A1**

(12) **EUROPEAN PATENT APPLICATION**

(43) Date of publication:
**07.08.2024 Bulletin 2024/32**

(21) Application number: **23154242.4**

(22) Date of filing: **31.01.2023**

(51) International Patent Classification (IPC):
***C12M 1/00*** (2006.01)     ***C12M 1/26*** (2006.01)
***C12M 1/34*** (2006.01)

(52) Cooperative Patent Classification (CPC):
**C12M 21/02; C12M 23/18; C12M 29/06;
C12M 33/10; C12M 41/12; C12M 41/26;
C12M 41/32; C12M 41/34**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Skyfarm Climate Technologies UG
10587 Berlin (DE)**

(72) Inventor: **FRIES, Luke
10587 Berlin (DE)**

(74) Representative: **Frick, Robert
Lorenz Seidler Gossel
Rechtsanwälte Patentanwälte
Partnerschaft mbB
Widenmayerstraße 23
80538 München (DE)**

(54) **SYSTEM AND METHOD FOR DIRECT ATMOSPHERIC CARBON CAPTURE AND MICROORGANISM CULTIVATION**

(57) The invention relates to a system and method for large scale direct atmospheric carbon capture (DAC) and microorganism, preferably microalgae cultivation. The system comprises an air pre-heating chamber, a bioreactor chamber containing a bulk liquid water pond containing microorganisms, which have the ability to sequester $CO_2$ dissolved in the water in their cells through the process of photosynthesis, preferably microalgae, and aerosolizers configured for aerosolizing water from the surface of the bulk liquid water, an air exhaust stack and a growth support system. The process comprises continuously causing ambient air to flow through the chambers, aerosolizing microorganism-containing water from the surface of the bulk liquid water pond at positions below the bioreactor inlet openings, and directing the continuous flow of heated air through the thus generated aerosol.

Figure 1

EP 4 410 943 A1

**Description**

TECHNICAL FIELD

The invention relates to a system and method for large scale direct atmospheric carbon capture (DAC) and microorganism, including microalgae cultivation.

BACKGROUND

To decrease the effects of global anthropogenic climate change, atmospheric $CO_2$ levels must be reduced as soon as possible. This means reducing $CO_2$ emissions as well as removing existing atmospheric $CO_2$. One method to remove existing atmospheric $CO_2$ is by stimulating the growth of organisms that sequester carbon in their cells through the process of photosynthesis.

Phytoplankton are a diverse set of autotrophic microscopic organisms present in fresh and saline water that form the basis of the aquatic food chain. They are an important part of the natural atmospheric carbon capture cycle, removing dissolved $CO_2$ from water bodies. Some members of this set, like many algae and cyanobacteria, can be cultivated deliberately, and currently are, for example, for the production of biofuels, fertilizers, livestock feed, and industrial or medicinal compounds. In general, these technologies rely on cultivating phytoplankton growing in liquid water, and the forms of these systems are defined by the need to contain and direct liquid water. The growth of phytoplankton is naturally constrained by three core requirements: essential micronutrients (iron, phosphate etc.), light, and dissolved $CO_2$. For different reasons, however, available state of the art cultivation systems are not suitable for direct atmospheric carbon capture (DAC), and suffer other drawbacks.

State of the art cultivation systems comprise conventional phytoplankton photobioreactors, which consist of an enclosed vessel that contains the liquid water growth medium. The water suspends an amount of cellular mass that is made to reproduce by controlling the input of dissolved $CO_2$, usually via mechanically pumping gaseous $CO_2$ through the water, by providing adequate light input via artificial lighting or otherwise, and by precisely controlling the concentration of nutrients dissolved in the liquid. While highly productive, such bioreactors are extremely energy intensive to operate due to the demanding energy requirements of artificial lighting, fluid circulation, and $CO_2$ input. In a variant called a photobioreactor array, an array of transparent pipes or tubes forms an extremely long bioreactor that allows for more efficient sunlight exposure to the liquid water within. These systems are commonly connected to industrial plants with $CO_2$ rich effluent gas, for example coal power stations. Although more efficient than standard bioreactors due to utilization of sunlight as opposed to artificial light, these systems still rely on forced $CO_2$ input and thus cannot directly capture atmospheric $CO_2$. They also have high energy requirements to circulate the liquid.

Raceway ponds are another contemporary algae cultivation technique. They are long artificial pools filled with water, using only the natural sunlight and dissolved $CO_2$ to grow cell mass, with nutrient inputs maintained by an operator. This method is cheap, but efficiency is constrained by the slow rate of natural $CO_2$ dissolution in surface water, and by large cultivation areas required. Thus, also this setup is not effective or practical for DAC. Also, typical cell densities are between 1-5 grams per liter, which then requires significant energy expenditure for harvesting and dewatering steps.

Thin Layer Cascade (TLC) systems form yet another contemporary cultivation technique. These are typically an open, inclined plane several meters in length, with a 1-3° slope. A thin 10-50 mm layer of growth medium flows over the surface. Growth media can be recycled by pumping from the low end to the high end via retention tanks, and water flow over the surface is induced by gravity. This arrangement allows rapid cycling of cells between darkness and illumination, thus allowing higher cell densities of up to 30 grams per liter, facilitates gas exchange due to the high surface area to volume ratio of the thin growth medium layer, and encourages optimal nutrient uptake due to the nature of media circulation. These systems are promising for algal cultivation, as they can be tightly controlled from a nutrient and illumination perspective. However, they still require significant energy usage as well as some amount of environmental control to mitigate the risks of open, non-sterile systems. The invention seeks to provide a system and method addressing these limitations of contemporary microalgae production technologies and achieving significant benefits both for microalgae cultivation and direct atmospheric carbon capture (DAC).

BRIEF DESCRIPTION OF THE INVENTION

Against this background, the invention proposes a system for direct atmospheric carbon capture and microorganism cultivation, comprising: a pre-heating chamber connected to the outside atmosphere by one or more system inlet openings; a bioreactor chamber connected to the pre-heating chamber by one or more bioreactor inlet openings, which open into the bioreactor chamber, wherein a lower section of the bioreactor chamber contains a bulk liquid water pond containing microorganisms, which have the ability to sequester $CO_2$ dissolved in the water in their cells through the process of photosynthesis, preferably microalgae, and wherein the bioreactor chamber comprises one or more aerosolizers arranged underneath the bioreactor inlet openings and configured for aerosolizing water from the bulk liquid water; an exhaust stack connected to the bioreactor chamber by a bioreactor outlet opening; and a growth support system, which is a circulation system for circulating the microorganism-containing water from and to the bulk liquid water pond, comprising a water reservoir connected to the bulk liquid water pond by a drain port and a feed port, a pump for circulating water from and to the bulk liquid water pond, one or more sensors for sensing process-relevant water parameter, one or more manipulation devices properties for manipulating process-relevant water parameters, and an apparatus for dewatering and microorganism harvesting.

A method according to the invention comprises the following steps: continuously causing ambient air to flow from the outside atmosphere through the system inlet openings into the pre-heating chamber; continuously heating the air inside the pre-heating chamber, preferably using solar energy, thereby generating an upwards flow of air inside the pre-heating chamber; continuously causing heated air to flow from the pre-heating chamber into the bioreactor chamber through the bioreactor inlet openings; aerosolizing, preferably continuously aerosolizing microorganism-containing water from the bulk liquid water pond at positions below the bioreactor inlet openings, and directing the continuous flow of heated air through the thus generated aerosol cloud; continuously causing an upward flow of air from the bioreactor chamber to the outward atmosphere through the exhaust stack.

DETAILED DESCRIPTION OF THE INVENTION

The invention relates to a system and method for direct atmospheric carbon capture and microorganism cultivation, as described above.

In one embodiment, the pre-heating (outer) chamber surrounds the bioreactor (inner) chamber, at least in part. Specifically, it is preferred that the side surfaces of the bioreactor chamber, or at least significant parts thereof are encircled by the pre-heating chamber. In an example, the bioreactor chamber may have a cylinder shape and the pre-heating chamber forms a mantle around the cylinder-shaped bioreactor chamber.

In one embodiment, the pre-heating chamber and the bioreactor chamber lie at the same level, and are usually ground built. In a preferred embodiment, the lower section of the bioreactor chamber is below grade and the level of the bulk liquid water pond therein is at grade, and the upper section of the inner chamber is built above that. The exhaust stack is preferably mounted on top of the bioreactor chamber. The bioreactor outlet opening is preferably located at the top of the bioreactor chamber.

Other embodiments of the invention comprise square or rectangular pre-heating chamber structures, which may be regular greenhouse structures, with a bioreactor chamber partition. In embodiments, one larger pre-heating chamber may comprise two or more bioreactor chambers distributed therein.

The volume ratio of the pre-heating chamber to the bioreactor chamber may be above 2:1, preferably above 2,5:1. The ratio of the height of the exhaust stack to the height of the bioreactor chamber may be above 2:1, preferably above 2,5:1. The ratio of the width of the bioreactor chamber to its height is an important parameter for system adaption to outside conditions and system efficiency. It can be between 1:1 and 1:2, for example. Preferred values can be between 1:1,4 and 1:1,8.

The pre-heating chamber functions as a solar radiation collector and a thermal buffer for the rest of the system. When collecting solar radiation, the air inside this chamber is heated and expands in volume. The positioning of the bioreactor inlet openings is such that expanded warm air will move into the bioreactor chamber, in turn drawing fresh outside air into the pre-heating chamber through the preferably multiple system inlet openings. Specifically, the bioreactor inlet openings are preferably distributed around an inner circumference of the pre-heating chamber, above the surface level of the bulk liquid water pond. The system inlet openings preferably open into a lower section of the pre-heating chamber, and are more preferably arranged at or close to its base. The cycle of air drawn through the system inlet openings, heated in the pre-heating chamber and further drawn into the bioreactor chamber, in operation of the system, will repeat as long as the temperature inside the pre-heating chamber is kept higher than the ambient temperature outside. This is preferably achieved by direct solar heating as well as from latent heat energy stored in the chamber material itself.

The bioreactor chamber acts as a bioreactor system. As explained above in the context of the pre-heating chamber, it has preferably multiple inlet openings distributed around its outer circumference, which connect the bioreactor chamber to the pre-heating chamber. Further, as also mentioned above already, there is a preferably single bioreactor outlet opening leading directly to the exhaust stack. The lower section of the bioreactor chamber contains bulk liquid water containing organisms that sequester carbon in their cells through the process of photosynthesis, preferably microalgae. The upper section of the bioreactor chamber is where incoming warm air, incoming through the bioreactor inlet openings from the pre-heating chamber, mixes with water aerosols. The aerosolizers, for example ultrasonic aerosolizers, are located underneath preferably each bioreactor inlet opening, thereby facilitating newly generated aerosols to be injected into the incoming airstream. The aerosolizers may be arranged at a surface level of the bulk liquid water pond. Different aerosolization techniques, that may be used in the inventive system, comprise installations that could be implemented at different heights inside the bioreactor chamber, for example pressurized spray nozzles.

In operation, air moves continuously through the pre-heating chamber, the bioreactor chamber and the exhaust stack. The pond at the base of the bioreactor chamber contains growing microalgae cells. This water is continuously circulated through an external growth-support system. The bioreactor chamber is where the mixing of air and water aerosols happens. Specifically, the incoming pre-heated air from the pre-heating chamber is mixed with liquid water aerosols generated at the pond surface. Incoming pre-heated air from the pre-heating chamber is preferably directed into the bioreactor chamber horizontally, or at an angle of 45° or less from horizontally, to meet with upstream aerosols at a right angle, or at least a 45° angle. The stream of incoming air is preferably a turbulent stream. Ideally, a turbulent mixing of the incoming airstream and the aerosol clouds is effected. Because the microalgae cells photosynthesize, the water is depleted of some amount of dissolved $CO_2$.

The air inside the bioreactor chamber is still relatively warmer than the external atmosphere, and in addition to new air being inserted from the pre-heating chamber, and it rises through the central outlet opening into the exhaust stack. Specifically, the system of the invention uses the stack effect to move large volumes of air efficiently and without significant energy input. A natural draft will form where the warmer air rises out of the system through the exhaust and cooler outside air is drawn in at the bottom. The airflow in the system of the invention can, in one embodiment, be controlled through the progressive opening and closing of internal apertures, to maintain an effective internal growth environment.

The exhaust stack preferably is a tall and vertical pipe, typically of cylindrical or slightly conical shape, that is connected directly to the bioreactor chamber outlet. The other end of the stack is open to the atmosphere. Due to the stack effect, warm air inside the pipe rises and accelerates until it escapes from the top of the pipe. This allows to model the overall volumetric airflow through the system as a function of the exhaust stack height, as well as internal and external air temperatures, among other things. Speaking generically, a taller stack will, to a certain extent, result in a higher volumetric flow rate.

Preferably, a condenser, like condenser vanes, fin arrays or similar, is arranged in the upper part of the bioreactor chamber and/or inside the exhaust stack. This will allow to recover water more efficiently.

The growth support system functions to monitor and replenish necessary nutrients for the target cultivar, monitor and balance process-relevant parameters, monitor and inform control systems about water temperature such that it can be kept within cultivar tolerance ranges, and perform autonomous harvesting and dewatering via continuous flow centrifugation, or a similar technique. The process-relevant parameters may comprise one or more of water temperature, pH, oxygen content, $CO_2$ content, nutrient content, and other chemicals content. The apparatus for dewatering and microorganism harvesting may comprise a continuous flow centrifugation device. As the growth support system performs multiple functions, it can be considered as a set of subsystems.

The purpose of the reservoir is to provide a fill source for the pond so as to maintain the correct water level, which would otherwise decrease due to aerosolization, evaporation and removal to the dewatering system. The relative water volumes of the reservoir and the pond can be less than 1,5:1, or less than 1:1. This ratio of the reservoir volume to pond volume is preferred to be as small as possible, as this decreases the time the microorganisms spend outside of the bioreactor chamber, and decreases the total mass of nutrients needed to maintain correct nutrient concentrations.

A basic implementation of a growth support system may resemble a water reservoir connected to the drain port of the bioreactor chamber via means such as a solid cone centrifuge, and pump, if necessary. Nutrient and other relevant sensors are installed inside this reservoir. Finally, there is a fill pump connected to the feed port of the bioreactor chamber.

As in the continuous growth system of the invention, only a small percentage of the total biomass is reproducing at a time (an exemplary value may be 10%), the apparatus for dewatering and microorganism harvesting should be configured such that, in operation, it removes only a small total percentage of the microorganisms that pass through it. The system can be operated in a way such as to have a bulk stock of cultivar, which contains several out of phase sub-cultures. Harvest can be done daily on only a part, for example on between 2% and 20% of the total biomass present. Total present biomass can be increased or decreased depending on the expected external conditions, for example during winter months the total biomass can be increased, as reproduction rate can be expected to fall.

The system inlet openings and/or the bioreactor inlet openings are, in a preferred embodiment, controllable between fully open, fully closed and, preferably, also partly closed positions.

DESCRIPTION OF THE DRAWINGS

Further details and advantages of the invention will be described in the following with reference to figures and examples. The figures show:

Figure 1:    an overview drawing of a system according to the invention;

Figure 2:    a schematic illustration of a pre-heating chamber of the system;

Figure 3:    a process diagram for the pre-heating chamber;

Figure 4:    a schematic illustration of a bioreactor chamber of the system;

Figure 5:    a process diagram for the bioreactor chamber;

Figure 6:    a schematic illustration of an exhaust stack of the system;

Figure 7:    a schematic illustration of a growth control system of the system; and

Figure 8:    a process overview for the biomass growth in the bioreactor chamber.

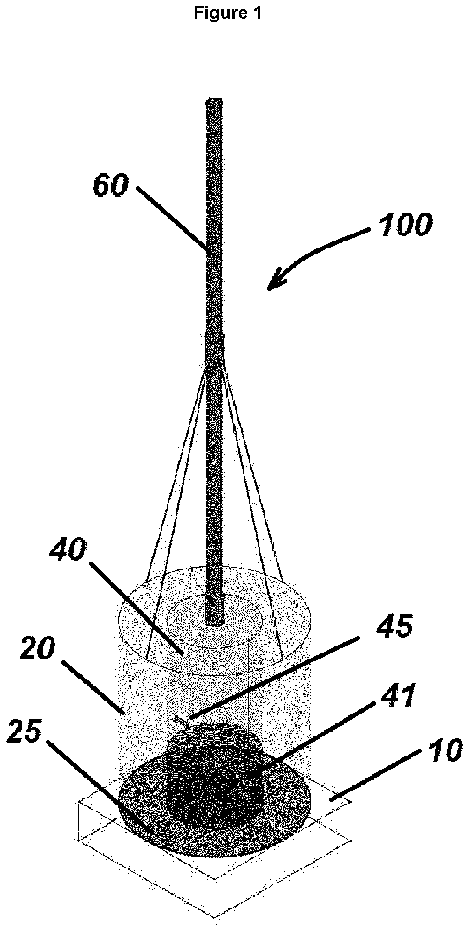

Figure 1 shows a schematic illustration of a system 100 for direct atmospheric carbon capture and microalgae cultivation, according to the invention. The system 100 comprises a pre-heating chamber 20 in the form of a cylinder mantle-shaped outer chamber, encircling a bioreactor chamber 40, which is in the form of a cylinder-shaped inner chamber. The pre-heating chamber 20 and the bioreactor chamber 40 lie at the same level and are built on floor slab 10. A growth control system 80, also forming part of the system 100, is not shown in Figure 1, but will be discussed later. The system 100 is in the form of a plant built on floor slab 10, forming a building of its own.

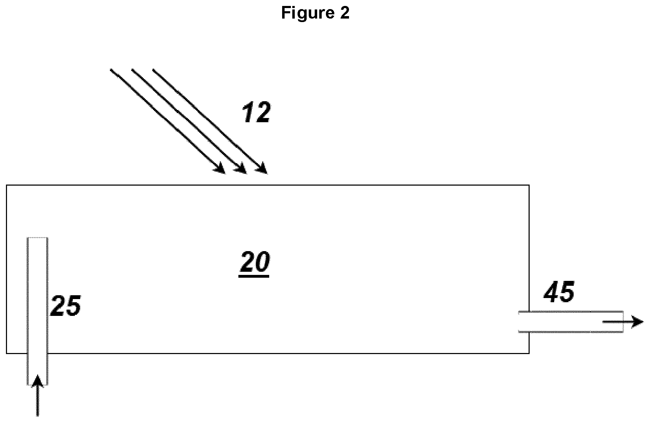

The pre-heating chamber 20 is connected to the outside atmosphere by system inlet openings 25, of which only one is shown in Figure 1 for illustrative purposes, but which can be more in practice, at the bottom of the pre-heating chamber 20. Figure 2, to complement the overview illustration of Figure 1, shows a schematic illustration of the pre-heating chamber 20.

The system inlet openings 25 are arranged at the base of the pre-heating chamber 20, and the bioreactor inlet openings 45, which are exit openings for the pre-heating chamber 20, are positioned at an upper portion of the pre-heating chamber 20. In operation, air is drawn through the system inlet openings 25, heated in the pre-heating chamber 20 and further flows out through the bioreactor inlet openings 25, into the bioreactor chamber 40. The heating of air in the pre-heating chamber 20 is achieved by direct solar heating 12 as well as from latent heat energy stored in the chamber material itself.

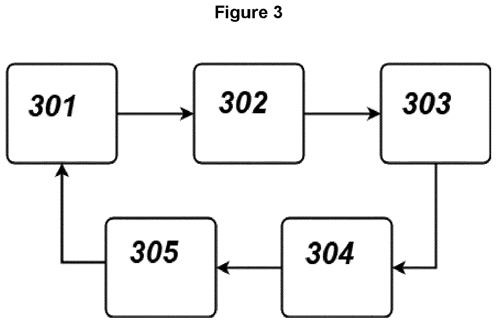

Figure 3 shows a process diagram for the continuous air flow through the pre-heating chamber 20. At step 301, cold air is drawn into the pre-heating chamber 20 through the system inlet openings 25. At step 302, solar radiation 12 and latent energy stored in the chamber walls heat the air inside the pre-heating chamber 20. At step 303, as a consequence to the warming, air volume increases and air density decreases, causing an upwards flow of the heated air inside the pre-heating chamber 20. At step 304, the warm air that has flown to the upper part of the pre-heating chamber 20 escapes the pre-heating chamber 20 through bioreactor inlet openings 45 and flows into the bioreactor chamber 40. The upwards flow of heated air at the same time, as illustrated at step 305, causes pressure in the lower part of the pre-heating chamber 20 to decrease, and air is drawn in (step 301). All steps 301-305 happen simultaneously, during operation of the system.

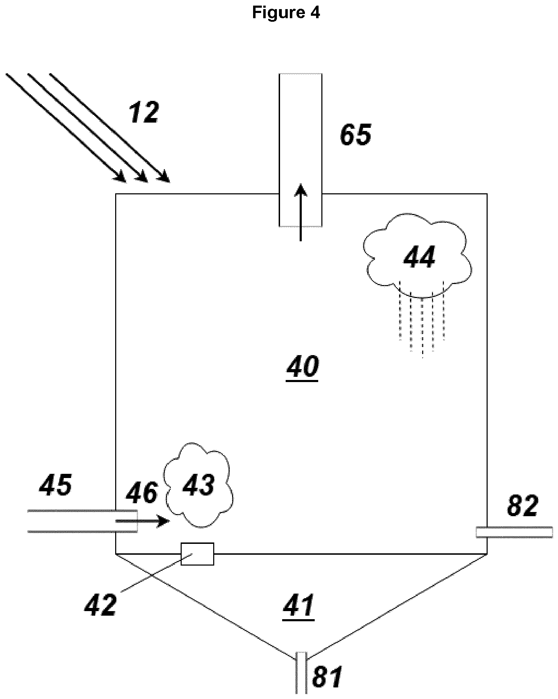

The bioreactor chamber 40 is connected to the pre-heating chamber 20 by bioreactor inlet openings 45, of which again only one is shown in Figure 1 for illustrative purposes, but which can be more in practice, distributed over the outer circumference of the cylinder-shaped bioreactor chamber 40. Figure 4, to complement the overview illustration of Figure 1, shows a schematic illustration of the bioreactor chamber 40.

The bioreactor inlet openings 45 are positioned at an upper portion of the pre-heating chamber 20, above the surface level of a bulk liquid water pond 41 occupying the lower portion of the bioreactor chamber 40. The water of the bulk liquid water pond 41 contains green microalgae, which have the ability to sequester $CO_2$ dissolved in the water in their cells through the process of photosynthesis.

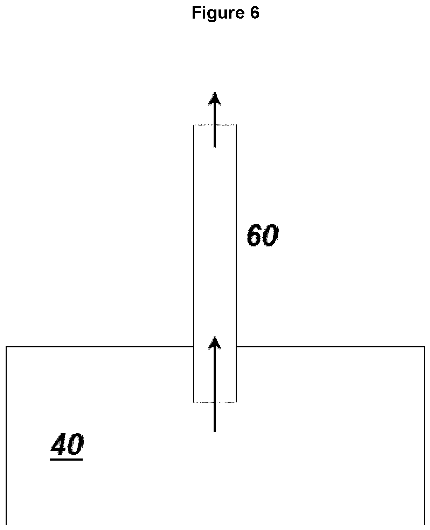

At the top of the bioreactor chamber 40 there is a bioreactor outlet opening 65, opening into an exhaust stack 60, explained in more detail later with reference to Figure 6, positioned at the center of the ceiling of the bioreactor chamber 40.

Ultrasonic transducers 42 acting as aerosolizers for microalgae-containing water from bulk liquid water pond 41, are located underneath each bioreactor inlet opening 45, thereby facilitating newly generated aerosols 43 to be injected into the incoming airstream 46. They are arranged at a surface level of the bulk liquid water pond 41. There is a turbulent mixing process between the incoming airstream and the aerosol clouds. Here, two airstreams, one the incoming air, which is preferably directed into the bioreactor chamber horizontally, and one the upwards stream of aerosol cloud, meet at a right angle.

The target size of the microscopic droplets of the aerosol may be as small as around 20 $\mu$m. Such aerosols, as can be appreciated by the square-cube law, have a very high surface area to volume ratio, and the system of the invention generates large effective water surface areas and facilitates rapid rates of gas transfer between the liquid growth medium and the atmosphere, with gas equilibration occurring very quickly.

Direct solar heating 12 promotes a further heating of air also in the bioreactor chamber 40. The air inside the bioreactor chamber 40 is still relatively warmer than the external atmosphere, and rises through the bioreactor outlet opening 65 into the exhaust stack 60.

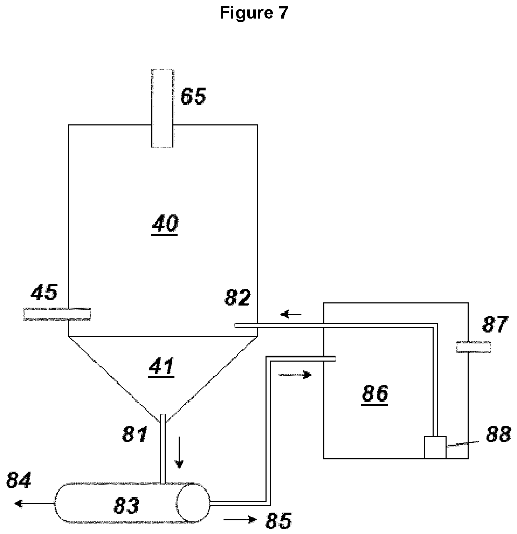

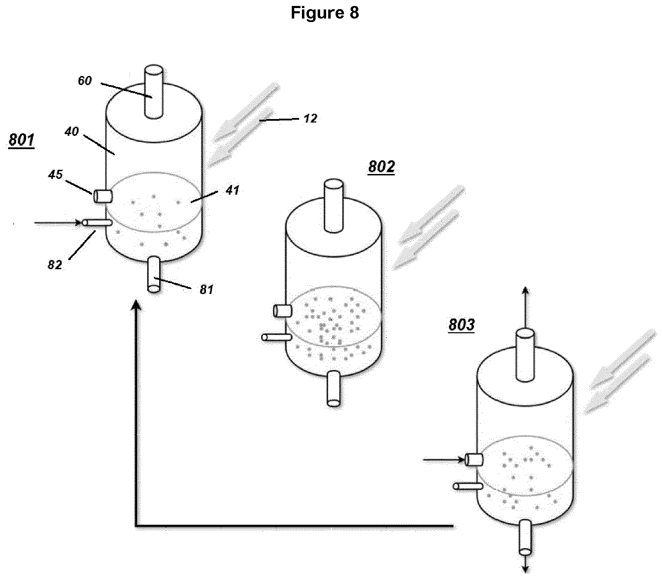

A drain port 81 at the lowest point of a conical bottom of the bioreactor chamber 40, which is for draining water from the bioreactor chamber 40 into the growth support system 80, later described with reference to Figures 7 and 8, and a feed port 82 for water from the growth support system 80, which laterally opens into the bioreactor chamber 40, are also visible in Figure 4.

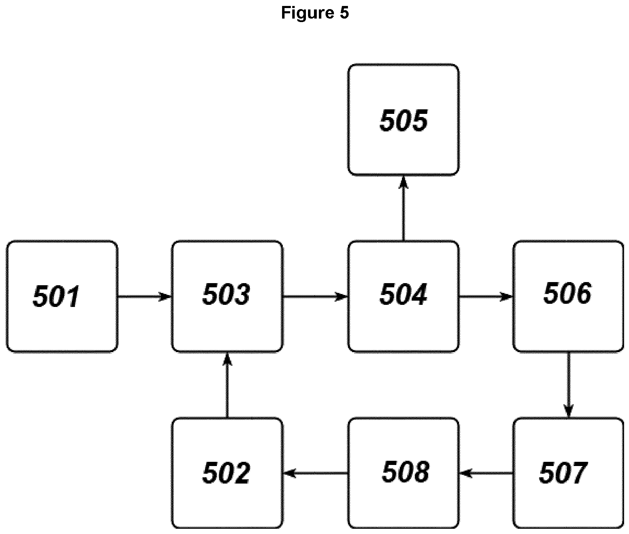

Figure 5 shows a process diagram for the airflow through the bioreactor chamber 40. At step 501, an airstream 46 from the pre-heating chamber 20 enters the bioreactor chamber 40. Simultaneously, aerosols 43 are generated from the bulk liquid water pond 41 by action of the aerosolizers 42, at step 502. There is then turbulent mixing of the inflowing air and the aerosol, at zones inside the bioreactor chamber 40 above the surface level of the liquid water pond 41, at step 503. $CO_2$ from the incoming airstream 46 is absorbed into the water aerosols 43, at step 504, and the incoming air hence efficiently loses some $CO_2$. At step 505, $CO_2$ depleted air rises through the bioreactor outlet opening 65 into the exhaust stack 60. Simultaneously, at step 506, aerosols 44 aggregate, and at step 507, rain down back into the pond 41. A condenser, like condenser vanes, fin arrays or similar, is arranged in the upper part of the bioreactor chamber to promote condensation and recover water efficiently. Because the microalgae cells photosynthesize, the water is then depleted of the previously dissolved $CO_2$, at step 508. All steps 501-508 happen simultaneously, during operation of the system.

The volume ratio of the pre-heating chamber 20 to the bioreactor chamber 40, in the given example, is 3:1. The ratio of the height of the exhaust stack 60 to the height of the cylindrical bioreactor chamber 40, in the given example, is also 3:1. The ratio of the width of the cylindrical bioreactor chamber 40 to its height, in the given example, is 1:1,6.

The pre-heating chamber 20 may completely be constructed from transparent polymer sheeting, for example polycarbonate sheeting of, for example, 1,0 to 3,0 mm thickness. The same applies to the bioreactor chamber 40. In preferred embodiments, at least the pre-heating chamber 20, but possibly also the bioreactor chamber 40, may have a transparent polymer sheeting, or glass, or insulated glasshouse roofing, while the outer walls are at least partially formed from different materials, and built from rammed earth, bricks, concrete, or the like. This applies to the base 10 as well. The walls and the base may serve as a thermal battery.

Yet further, the system 100 comprises an exhaust stack 60 connected to the bioreactor chamber 40. Specifically, the exhaust stack 60 is mounted on top of the bioreactor chamber 40. Figure 6 shows a schematic illustration of the exhaust stack 60. The exhaust stack 60 is a tall and vertical pipe. The far end is open to the atmosphere.

Figure 7 is a schematic illustration of a growth control system 80, which forms part of the system and is connected to the bioreactor chamber 40 over drain ports 81 and feed ports 82 (Figure 4).

The growth support system 80 functions to monitor and replenish necessary nutrients, monitor and balance process-relevant parameters, and perform harvesting and dewatering via continuous flow centrifugation.

A centrifuge 83 for autonomous harvesting and dewatering water, via continuous flow centrifugation, coming out of the pond 41 through the drain port 81 is located downstream the drain port 81. Dewatered biomass 84 exits the system. The biomass-depleted water 85 enters a water reservoir 86, where sensors (not shown) measure water temperature, pH, oxygen content, $CO_2$ content, and nutrient content. Additives are added at port 87. A fill pump 88 pumps biomass-depleted and nutrient-enriched water back into the pond 41 through feed port 82.

Figure 8 shows a process overview for the biomass growth in the bioreactor chamber 40. At step 801, biomass-depleted and nutrient-enriched water enters the pond 41 inside the bioreactor chamber 40 through feed port 82. At step 802, biomass reproduces by mitosis. At step 803, the water absorbs $CO_2$ from the incoming fresh air 46. Water is depleted of the dissolved $CO_2$ through photosynthesis. The biomassenriched water exits through the drain port 81. $CO_2$-depleted air exits through the exhaust stack 60. During the process, heat energy is supplied by sunlight 12.

EXAMPLE

The following example shows how much biomass can be produced, and how much $CO_2$ can be captured from ambient air, by a system and method of the invention.

To produce an average of 1 kg of biomass per day, at a typical PBR algae concentration of 1g/L, and assuming a 10% daily harvest volume (10% of total bio stock reproduces per day), 10,000L of growth media need to be maintained inside one reactor to realize a daily harvest of 1000g. 1kg of biomass requires -1.8kg of $CO_2$ to produce, so that means that the reactor should process at least 2400 cubic meters of air per day. Assuming a mixing / $CO_2$ capture efficiency of 10%, 24,000 cubic metres of air must be processed.

With these yield calculations, machine dimensions for a given geographic location can be designed as follows.

As a core factor in determining the volumetric flow rate through a climate engine is the local air temperature.

Additionally, the difference in average sunlight hours during each season is to be taken into account, because this affects the length of time it will take for the algae cells to reproduce naturally (assuming a cumulative 36 hours of illumination are required for mitosis). For the example herein, Berlin, Germany is assumed as a target installation location of the system. The historic climate averages shown in Table 1 were assumed for this location.

Table 1

| Season | Avg. Min. Temp. (°C) | Avg. Max. Temp. (°C) | Avg. Sunlight (hours/day) |
|---|---|---|---|
| Summer | 12,6 | 23,3 | 7,5 |
| Autumn | 6,0 | 13,3 | 5 |
| Winter | -1,3 | 4 | 1,6 |
| Spring | 4,3 | 14,0 | 4 |

At this target location, the severely restricted sunlight hours during winter mean that the bulk of the production should occur in summer. The system should hence be built large enough to produce sufficiently during summer to cover the winter deficit. Artificial illumination may be eventually used to prolong the daily growth window during winter. Given that a summer production should be roughly double the target daily yield to cover for the restricted winter production, that then requires that a system installed in Berlin should be capable of processing double the volume of air determined above ($2*24,000 = 48,000$ $m^3$). A design should hence aim to move 48000 $m^3$ during 7,5 hours of summer illumination, which gives a required maximum rate of 6400 $m^3$/hour, or 1.7 $m^3$/second.

A target cultivar strain C. Vulgaris can tolerate temperatures up to ~45°C. Hence, the air temperature inside the system 100 should not exceed this. The internal air temperature can be controlled by the material properties of the system 100 chamber's 20, 40 cover, for example a polycarbonate greenhouse sheeting, as well as by manually controlling air intake speed to allow air more or less time to heat up in the pre-heating chamber 20. For the following calculations, internal air temperature is assumed to be a constant 10°C higher than the external environmental air. Some further assumptions are made regarding the diameters of the air intake(s) 25, 45 and exhaust stack 60. For simplicity, the model matches the intake and exhaust diameters to 1,0 meters.

Using the Stack Effect volumetric airflow equation

$$q = \pi \, d_h^2 \, /4 \, [(2 \, g \, (\rho_o - \rho_r) \, h \,) \, / \, ( \, \lambda \, l \, \rho_r \, / \, dh + \Sigma\xi \, \rho_r \,)]^{\frac{1}{2}}$$

where:

$q$ = air vol rate ($m^3$/s)
$d_h$ = hydraulic diameter of exhaust
$g$ = acc. due to gravity (9,81 $m/s^2$)
$p_o$ =air density outside
$p_r$ = air density inside
$h$ = exhaust stack height (meters)
$\Sigma\xi$ = minor loss coefficient (1)

the required stack height for our maximum airflow requirement can be estimated as follows:

Total height: 8,5 m
Exhaust height: 5 m
Bioreactor chamber height: 3.5 m
Bioreactor chamber radius: 1,1 m
Bioreactor chamber volume: 5,6 $m^3$
pre-heating chamber height: 2 m
pre-heating chamber radius = 2,1 m
pre-heating chamber volume = 16,7 $m^3$ (22,3 $m^3$ incl. bioreactor)

The bioreactor chamber 40 airspace can be filled with aerosols at 5000:1 ratio air/water. 11L water containing 110g biomass then form around 33 billion aerosols having around 170 $m^2$ effective surface area.

**Claims**

1. System for direct atmospheric carbon capture and microorganism cultivation, comprising:

   a pre-heating chamber connected to the outside atmosphere by one or more system inlet openings;
   a bioreactor chamber connected to the pre-heating chamber by one or more bioreactor inlet openings, which open into the bioreactor chamber, wherein a lower section of the bioreactor chamber contains a bulk liquid water pond containing microorganisms, which have the ability to sequester $CO_2$ dissolved in the water in their cells through the process of photosynthesis, preferably microalgae, and wherein the bioreactor chamber comprises one or more aerosolizers arranged underneath the bioreactor inlet openings and configured for aerosolizing water from the bulk liquid water;
   an exhaust stack connected to the bioreactor chamber by a bioreactor outlet opening; and
   a growth support system, which is a circulation system for circulating the microorganism-containing water from and to the bulk liquid water pond, comprising a water reservoir connected to the bulk liquid water pond by a drain port and a feed port, a pump for circulating water from and to the bulk liquid water pond, one or more sensors for sensing process-relevant water parameter, one or more manipulation devices properties for manipulating process-relevant water parameters, and an apparatus for dewatering and microorganism harvesting.

2. The system of claim 1, wherein the pre-heating chamber at least in part surrounds the bioreactor chamber.

3. The system of any preceding claim, wherein the lower section of the bioreactor chamber is below the level of the pre-heating chamber, and the level of the bulk liquid water pond is at grade with the bottom of the pre-heating chamber.

4. The system of any preceding claim, wherein the exhaust stack is mounted on top of the bioreactor chamber, and is a tall and vertical pipe that is connected directly to the bioreactor chamber outlet, its other end open to the atmosphere.

5. The system of any preceding claim, wherein

   the volume ratio of the pre-heating chamber to the bioreactor chamber is above 2:1, preferably above 2,5:1; and/or
   the ratio of the height of the exhaust stack to the height of the bioreactor chamber is above 2:1, preferably above 2,5:1; and/or
   the ratio of the width of the bioreactor chamber is between 1:1 and 1:2, preferably between 1:1,4 and 1:1,8.

6. The system of any preceding claim, wherein

   the system inlet openings are arranged at or close to the base of the pre-heating chamber and open into a lower section thereof; and/or
   wherein the bioreactor inlet openings are distributed around an inner circumference of the pre-heating chamber.

7. The system of any preceding claim, wherein the aerosolizers are selected from the group of ultrasonic aerosolizers, preferably arranged at a surface level of the bulk liquid water pond, and pressurized spray nozzles, preferably arranged above the level of the liquid water pond.

8. The system of any preceding claim, wherein the process-relevant parameters measured and potentially influenced at the growth support system comprise one or more of water temperature, pH, oxygen content, $CO_2$ content, nutrient content, and other chemicals content.

9. The system of any preceding claim, wherein the growth support system comprises, as the apparatus for dewatering and microorganism harvesting, a continuous flow centrifugation device.

10. The system of any preceding claim, wherein a condenser is arranged in the upper part of the bioreactor chamber and/or inside the exhaust stack.

11. The system of any preceding claim, wherein the system inlet openings and/or the bioreactor inlet openings are controllable between fully open, fully closed and, preferably, also partly closed positions.

12. A method for direct atmospheric carbon capture and microalgae cultivation, using a system of any preceding claim, and comprising the following steps:

continuously causing ambient air to flow from the outside atmosphere through the system inlet openings into the pre-heating chamber;

continuously heating the air inside the pre-heating chamber, preferably using solar energy, thereby generating an upwards flow of air inside the pre-heating chamber;

continuously causing heated air to flow from the pre-heating chamber into the bioreactor chamber through the bioreactor inlet openings;

aerosolizing water from the bulk liquid water pond at positions below the bioreactor inlet openings, and directing the continuous flow of heated air through the thus generated aerosol cloud;

continuously causing an upward flow of air from the bioreactor chamber to the outward atmosphere through the exhaust stack.

13. The method of claim 12, further comprising:

circulating the microorganism-containing water from and to the bulk liquid water pond through the growth support system;

sensing process-relevant water parameters of the water in the growth support system;

if necessary, manipulating process-relevant water parameters of the water in the growth support system.

14. The method of claim 12 or 13, further comprising:

circulating the microorganism-containing water from and to the bulk liquid water pond through the growth support system;

continuously or discontinuously dewatering and harvesting microorganisms from the water in the growth support system.

15. Use of a system of any one of claims 1-11 for direct atmospheric carbon capture and microalgae cultivation.

# Figure 1

**Figure 2**

**Figure 3**

**Figure 4**

**Figure 5**

**Figure 6**

## Figure 7

# Figure 8

Europäisches Patentamt
European Patent Office
Office européen des brevets

# EUROPEAN SEARCH REPORT

Application Number

**EP 23 15 4242**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2022/389357 A1 (HUSCHEK GERD [DE] ET AL) 8 December 2022 (2022-12-08) * paragraphs [0039], [0059], [0060], [0061]; claims 1,8 * ----- | 1-15 | INV. C12M1/00 C12M1/26 C12M1/34 |
| Y | CN 112 779 138 A (GANSU KAIYUAN BIOTECHNOLOGY DEV CENTER) 11 May 2021 (2021-05-11) * claim 1; figure 1 * ----- | 1-15 | |
| A | WO 2012/100093 A2 (ALGAE AQUA CULTURE TECHNOLOGY INC [US] ET AL.) 26 July 2012 (2012-07-26) * claims 1, 17, 25, 26, 32 * ----- | 1-15 | |
| A | US 2009/221057 A1 (KENNEDY JAMES C [US]) 3 September 2009 (2009-09-03) * figure 1 * ----- | 1-15 | |
| A | CN 112 625 891 A (UNIV SHENZHEN) 9 April 2021 (2021-04-09) * figure 1 * ----- | 1-15 | TECHNICAL FIELDS SEARCHED (IPC) C12M |

The present search report has been drawn up for all claims

| Place of search | Date of completion of the search | Examiner |
|---|---|---|
| The Hague | 8 June 2023 | Guimarães, Bárbara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 4242

This annex lists the patent family members relating to the patent documents cited in  the above-mentioned European search report.
The members are as contained in the European Patent Office EDP file on
The European Patent Office is in no way liable for these particulars which are merely given for the purpose of information.

08-06-2023

| Patent document cited in search report | | Publication date | Patent family member(s) | | Publication date |
|---|---|---|---|---|---|
| US 2022389357 | A1 | 08-12-2022 | AU 2020381134 | A1 | 12-05-2022 |
| | | | CA 3155359 | A1 | 14-05-2021 |
| | | | CN 114616315 | A | 10-06-2022 |
| | | | DE 102019130109 | B3 | 11-03-2021 |
| | | | EP 4055135 | A1 | 14-09-2022 |
| | | | JP 2023500201 | A | 05-01-2023 |
| | | | US 2022389357 | A1 | 08-12-2022 |
| | | | WO 2021089085 | A1 | 14-05-2021 |
| CN 112779138 | A | 11-05-2021 | NONE | | |
| WO 2012100093 | A2 | 26-07-2012 | AU 2012207199 | A1 | 29-08-2013 |
| | | | BR 112013018402 | A2 | 11-10-2016 |
| | | | CA 2853605 | A1 | 26-07-2012 |
| | | | CN 103429362 | A | 04-12-2013 |
| | | | EP 2699363 | A2 | 26-02-2014 |
| | | | JP 2014509252 | A | 17-04-2014 |
| | | | RU 2013138450 | A | 27-02-2015 |
| | | | US 2012208254 | A1 | 16-08-2012 |
| | | | WO 2012100093 | A2 | 26-07-2012 |
| US 2009221057 | A1 | 03-09-2009 | NONE | | |
| CN 112625891 | A | 09-04-2021 | NONE | | |

EPO FORM P0459

For more details about this annex : see Official Journal of the European Patent Office, No. 12/82